(19) Europäisches Patentamt
European Patent Office
Office européen des brevets

(11) EP 0 749 746 B1

(12) **FASCICULE DE BREVET EUROPEEN**

(45) Date de publication et mention
de la délivrance du brevet:
**03.09.1997 Bulletin 1997/36**

(51) Int. Cl.⁶: **A61K 7/02**, A61K 7/027,
A61K 7/48, A61K 7/00,
A61K 7/032

(21) Numéro de dépôt: 96401313.0

(22) Date de dépôt: 17.06.1996

(54) **Composition cosmétique comprenant une dispersion de particule de polymère**

Kosmetisches Mittel enthaltend eine Dispersion von Polymerenteilchen

Cosmetic composition containing a dispersion of polymer particles

(84) Etats contractants désignés:
**AT BE CH DE DK ES FI FR GB GR IE IT LI LU MC NL PT SE**

(30) Priorité: **21.06.1995 FR 9507432**
**21.06.1995 FR 9507430**
**21.06.1995 FR 9507431**
**21.06.1995 FR 9507429**

(43) Date de publication de la demande:
**27.12.1996 Bulletin 1996/52**

(73) Titulaire: **L'OREAL**
**75008 Paris (FR)**

(72) Inventeurs:
• **Mougin, Nathalie**
**75011 Paris (FR)**
• **Mondet, Jean**
**93600 Aulnay sous Bois (FR)**
• **Bara, Isabelle**
**75013 Paris (FR)**

(74) Mandataire: **Dodin, Catherine**
**L'Oreal-D.P.I.,**
**90, rue du Général Roguet**
**92583 Clichy Cédex (FR)**

(56) Documents cités:
**EP-A- 0 195 575**      **EP-A- 0 409 690**
**EP-A- 0 447 286**      **EP-A- 0 486 394**
**EP-A- 0 497 144**      **EP-A- 0 502 769**
**WO-A-95/09874**      **GB-A- 1 202 796**

• **CHEMICAL ABSTRACTS, vol. 89, no. 22, 27 Novembre 1978 Columbus, Ohio, US; abstract no. 185917, XP002013897 & JP-A-53 094 041 (KOBAYASHI KOSE CO)**

## Description

La présente invention a trait à une composition cosmétique, dermatologique, pharmaceutique ou hygiénique, notamment pour le soin et/ou le maquillage de la peau pouvant se présenter sous forme de produit coulé, de poudre compacte ou de toute autre forme acceptable cosmétiquement, et comprenant une dispersion de particules de polymère dans un corps gras liquide, ainsi qu'à l'utilisation d'une telle dispersion dans des compositions cosmétiques.

Les compositions cosmétiques ou dermatologiques pouvant être appliquées sur la peau ou les lèvres comme produit de maquillage ou de soin, telles que les bases ou rouges pour lèvres ou les fonds de teint par exemple, contiennent généralement des corps gras tels que des cires et des huiles, des pigments et/ou charges et, éventuellement, des additifs. Il est connu que plus la quantité de cires présente dans la composition est importante, plus celle-ci a une consistance ferme, ce qui permet son utilisation sous forme de bâton.

Il est également connu des compositions se présentant sous forme de pâte souple, applicable à l'aide d'un pinceau par exemple. Ces compositions contiennent généralement une faible quantité de cires, et des corps gras de type pâteux et/ou huileux. Les corps gras de type pâteux sont généralement présents en quantité importante, de manière à obtenir une composition de consistance et de viscosité adéquates en permettant l'application.

On a toutefois constaté que ces compositions présentaient une certaine capacité à migrer, c'est-à-dire avaient tendance à se propager à l'intérieur des ridules de la peau, notamment celles qui entourent les lèvres, en créant un effet inesthétique, cette migration étant en partie due à la présence de corps gras huileux ou pâteux.

Il subsiste donc le besoin d'une composition qui migre peu après son application sur la peau et au cours du temps.

La demanderesse a constaté que, de façon tout à fait surprenante, l'utilisation d'une dispersion selon l'invention dans une composition cosmétique, dermatologique, pharmaceutique ou hygiénique pouvait permettre d'obtenir un film de très bonne tenue, qui migre peu au cours du temps, tout en étant très agréable à porter. Ceci est en particulier le cas lorsque le polymère en dispersion est un polymère non filmogène, en particulier réticulé.

D'autre part, on a constaté que certaines compositions selon l'invention permettent d'estomper de manière convenable, par camouflage, les ridules de la peau.

En effet, les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. Il est usuel de combattre l'apparition des ridules en traitant lesdites rides et ridules à l'aide de produits cosmétiques contenant des actifs agissant sur la peau, par exemple en l'hydratant ou en améliorant son renouvellement cellulaire ou encore en favorisant la synthèse du collagène qui compose le tissu cutané. Une autre possibilité consiste à masquer ou camoufler les ridules déjà formées, par exemple à l'aide de produits de maquillage tels que des fonds de teint ou des crèmes teintées.

Toutefois, la demanderesse a constaté que, de façon surprenante et inattendue, l'utilisation d'une dispersion dans un corps gras liquide de particules de polymère non filmogène et stabilisé en surface, dans une composition à appliquer sur la peau, pouvait permettre d'améliorer le 'camouflage' des rides et ridules déjà formées.

Sans être tenu par cette explication, on peut considérer que lors de l'application de la composition, les particules de polymères en dispersion dans le corps gras vont pénétrer à l'intérieur des ridules de la peau et donc les camoufler lorsque le visage est au repos. Lorsque la peau va se déformer, par exemple lors des mouvements du visage, la dispersion des particules de polymère, qui ne sont pas filmogènes, va suivre les mouvements du visage. Le corps gras de la dispersion, qui a notamment pour fonction de lier les particules entre elles, ainsi que de les lier sur les bords de la ridule, également appelés 'lèvres' de la ridule, va assurer, lors des mouvements du visage, l'adhésion et la cohésion entre les particules de polymère et les lèvres de la ridule, d'où également un certain estompage desdites ridules lorsque le visage est en mouvement.

Il est à noter que l'utilisation d'une dispersion dans une huile d'un polymère entièrement filmogène ne conviendrait pas, étant donné que le film obtenu par coalescence des particules ne pourrait pas se déformer, lors des mouvements du visage, de manière suffisamment aisée et rapide afin d'obtenir l'effet recherché.

D'autre part, l'utilisation d'une dispersion aqueuse d'un polymère non filmogène ne conviendrait également pas. En effet, dans ce cas, on constate une absorption de la majeure partie de l'eau contenue dans la dispersion par la peau. Il s'en suit que les particules de polymère ne sont plus liées entre elles et/ou avec les lèvres de la ridule, et ne permettent donc plus d'obtenir l'effet recherché.

Dans le cas des dispersions selon l'invention, il est possible qu'une petite partie du corps gras soit absorbée par la peau. Toutefois, la quantité restante dans la dispersion est suffisante pour permettre une bonne cohésion et adhérence, et donc un estompage adéquat des ridules.

D'autre part, la demanderesse a constaté que, de façon surprenante et inattendue, l'utilisation d'une dispersion dans une huile, de particules sphériques de polymères stabilisés en surface, dans une composition de maquillage pour les yeux, pouvait permettre d'allonger et de gainer de manière particulièrement remarquable les cils et conférer à la composition une bonne rémanence à l'eau.

Ces compositions ont pour but d'embellir les cils en y déposant un film qui peut les embellir, les colorer, les allonger et/ou les amplifier, éventuellement en les protégeant et/ou en les traitant.

Enfin, la demanderesse a constaté que, de façon surprenante et inattendue, l'utilisation d'une dispersion dans une huile, de particules sphériques de polymères stabilisés en surface, pouvait permettre d'améliorer le compactage des compositions comprenant une quantité importante de composés pulvérulents.

En effet, on connaît des compositions, notamment cosmétiques, dermatologiques, pharmaceutiques ou hygiéniques qui peuvent se présenter sous forme d'une poudre dite compacte, obtenue par compactage. Il s'agit généralement de compositions anhydres pouvant être constituées principalement de particules solides et d'un liant gras, mises en forme par compression. On peut en particulier citer les fards à paupières ou les fards à joues. L'élaboration de telles compositions soulève toutefois de nombreuses difficultés car la composition finale doit être suffisamment homogène et compacte pour présenter une bonne aptitude au prélèvement et pour éviter par ailleurs une fragmentation pouvant être provoquée notamment par des chocs.

Grâce à l'utilisation d'une composition selon l'invention, on peut obtenir une composition se présentant sous forme d'une poudre compacte, stable au stockage et présentant une bonne cohésion, ce qui permet d'éviter un effritement facile du produit compacté.

La présente invention a donc pour objet une composition cosmétique, comprenant des corps gras et des composés pulvérulents, caractérisée par le fait qu'elle comprend une dispersion de particules de polymère stabilisé en surface dans un corps gras liquide.

Elle a également pour objet une composition se présentant sous forme d'un produit coulé et comprenant au moins une cire, caractérisée par le fait qu'elle comprend en outre une dispersion de particules de polymère réticulé et stabilisé en surface, dans un corps gras liquide cosmétiquement, dermatologiquement, hygiéniquement ou pharmaceutiquement acceptable.

Elle a également pour objet une composition se présentant sous forme d'une poudre compacte, comprenant un liant gras et des composés pulvérulents, caractérisée par le fait qu'elle comprend une dispersion de particules de polymère stabilisé en surface dans un corps gras liquide cosmétiquement, dermatologiquement, pharmaceutiquement ou hygiéniquement acceptable.

Elle a également pour objet une composition comprenant une dispersion de particules de polymère non filmogène et stabilisé en surface, dans un corps gras liquide non volatil et cosmétiquement, dermatologiquement, hygiéniquement ou pharmaceutiquement acceptable, ladite dispersion ayant un taux de matière sèche d'au moins 15% en poids.

Un autre objet de l'invention est l'utilisation dans une composition cosmétique pour le maquillage des yeux, comprenant des corps gras et des composés pulvérulents, d'une dispersion de particules de polymère stabilisé en surface dans un corps gras liquide, afin d'améliorer l'allongement des cils et/ou d'améliorer la rémanence à l'eau de la composition.

Un autre objet est l'utilisation dans une composition sous forme de produit coulé et comprenant au moins une cire, d'une dispersion de particules de polymère non filmogène et stabilisé en surface dans un corps gras liquide cosmétiquement, dermatologiquement, pharmaceutiquement ou hygiéniquement acceptable, dans le but d'atténuer la migration des constituants de la composition dans les ridules de la peau et/ou dans le but d'améliorer la tenue de la composition.

Un autre objet est l'utilisation dans une composition se présentant sous forme d'une poudre compacte, comprenant un liant gras et des composés pulvérulents, d'une dispersion de particules de polymère stabilisé en surface dans un corps gras liquide cosmétiquement, dermatologiquement, pharmaceutiquement ou hygiéniquement acceptable, dans le but de faciliter le compactage de ladite composition.

Un autre objet est l'utilisation dans une composition cosmétique, dermatologique, pharmaceutique ou hygiénique, d'une dispersion de particules de polymère non filmogène et stabilisé en surface, dans un corps gras liquide non volatil et cosmétiquement, dermatologiquement, hygiéniquement ou pharmaceutiquement acceptable, ladite dispersion ayant un taux de matière sèche d'au moins 15% en poids, dans le but d'estomper les rides et/ou les ridules de la peau.

Un avantage de l'utilisation d'une dispersion de particules selon l'invention est que les particules restent à l'état de particules élémentaires, sans former d'agglomérats, dans le corps gras, ce qui ne serait pas le cas avec des particules minérales de taille nanométrique.

Encore un autre avantage d'une telle dispersion est qu'il est possible de calibrer à volonté la taille des particules de polymère, et de moduler leur polydispersité en taille lors de la synthèse. Il est ainsi possible d'obtenir des particules de très petite taille, qui sont invisibles à l'oeil lorsqu'elles sont dans la composition et lorsqu'elles sont appliquées sur la peau. Ceci ne serait pas possible avec des pigments sous forme particulaire, leur constitution ne permettant pas de moduler la taille moyenne des particules.

On a de plus constaté que les compositions selon l'invention à étaler sur la peau, les semi-muqueuses ou les muqueuses, présentent des qualités d'étalement et d'adhésion à la peau particulièrement intéressantes, ainsi qu'un toucher onctueux et agréable.

Les compositions selon l'invention comprennent donc une dispersion stable de particules généralement sphériques d'au moins un polymère stabilisé en surface, dans un corps gras liquide cosmétiquement et/ou dermatologiquement acceptable.

Ces dispersions peuvent notamment se présenter sous forme de nanoparticules de polymères en dispersion stable dans ledit corps gras. Les nanoparticules sont de préférence d'une taille comprise entre 5 et 600 nm, étant donné qu'au-delà d'environ 600 nm, les dispersions de particules deviennent beaucoup moins stables.

Les polymères utilisés dans la présente demande peuvent être de toute nature.

On peut ainsi employer des polymères radicalaires, des polycondensats, voire des polymères d'origine naturelle. Le polymère peut être choisi par l'homme du métier en fonction de ses propriétés, selon l'application ultérieure souhaitée pour la composition.

Ainsi, le polymère peut être filmogène ou non filmogène; dans ce second cas, il peut en particulier se présenter sous la forme d'un polymère réticulé.

Il est donc possible d'utiliser des polymères filmogènes, de préférence ayant une température de transition vitreuse (Tg) basse, inférieure ou égale à la température ambiante. On obtient ainsi une dispersion qui peut filmifier lorsqu'elle est appliquée sur un support, ce qui n'est pas le cas lorsque l'on utilise des dispersions de pigments minéraux selon l'art antérieur.

Il est également possible d'utiliser des polymères non filmogènes, éventuellement réticulés, qui pourront être utilisés en tant que charges dispersées de manière stable dans une huile. On réticule le polymère utilisé de manière à empêcher sa filmification lorsqu'il est déposé sur la peau. La réticulation peut être effectuée par tout moyen connu de l'homme du métier. L'agent réticulant peut être choisi parmi tout agent réticulant connu dans la polymérisation radicalaire, en particulier les réticulants di- ou multifonctionnels tels que le diméthacrylate d'éthylène-glycol ou le divinylbenzène.

Les polymères utilisables dans le cadre de la présente invention ont de préférence un poids moléculaire de l'ordre de 2000 à 10 000 000, et une température de transition vitreuse de -100°C à 300°C.

Lorsque le polymère présente une Tg trop élevée pour l'application souhaitée, on peut lui associer un plastifiant de manière à abaisser la Tg du mélange utilisé. Le plastifiant peut être choisi parmi les plastifiants usuellement utilisés dans le domaine d'application et notamment parmi les composés susceptibles d'être des solvants du polymère.

Parmi les polymères filmogènes non réticulés, on peut citer des homopolymères ou des copolymères radicalaires, acryliques ou vinyliques, de préférence ayant une Tg inférieure ou égale à 30°C.

Parmi les polymères non filmogènes, on peut citer des homopolymères ou copolymères radicalaires, vinyliques ou acryliques, éventuellement réticulés, ayant de préférence une Tg supérieure ou égale à 40°C, tels que le polyméthacrylate de méthyle, le polystyrène ou le polyacrylate de tertiobutyle.

Le corps gras liquide dans lequel sont dispersées les particules de polymère, peut être constitué de toute huile cosmétique ou dermatologiquement acceptable, notamment choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

Par corps gras liquide, on entend tout milieu non aqueux liquide à température ambiante.

On peut ainsi citer les huiles hydrocarbonées telles que l'huile de paraffine ou de vaseline; l'huile de vison, l'huile de tortue, l'huile de soja, le perhydrosqualène; l'huile d'amande douce, de calophyllum, de palme, l'huile de pépins de raisin, l'huile de sésame, l'huile de maïs, l'huile de colza, l'huile de tournesol, l'huile de coton, de ricin, d'avocat, de jojoba, d'olive ou de germes de céréales; des esters d'acide lanolique, d'acide oléique, d'acide laurique, d'acide stéarique; les esters gras, tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyldécyle, le myristate ou le lactate de 2-octyl-dodécyle, le succinate de 2-diéthylhexyle, le malate de diisostéaryle, le triisostéarate de glycérine ou de diglycérine; les acides gras supérieurs tels que l'acide myristique, l'acide palmitique, l'acide stéarique, l'acide béhénique, l'acide oléique, l'acide linoléique, l'acide linolénique ou l'acide isostéarique; les alcools gras supérieurs tels que le cétanol, l'alcool stéarylique ou l'alcool oléique, l'alcool linoléique ou linolénique, l'alcool isostéarique ou l'octyl dodécanol; les huiles siliconées telles que les PDMS, éventuellement phénylées telles que les phényltriméthicones ou éventuellement substitués par des groupements aliphatiques et/ou aromatiques, éventuellement fluorés, ou par des groupements fonctionnels tels que des groupements hydroxyles, thiols et/ou amines.; les polysiloxanes modifiés par des acides gras, des alcools gras ou des polyoxyalkylènes, les silicones fluorées, les huiles perfluorées. On peut également utiliser des huiles volatiles, telles que la cyclotétradiméthylsiloxane, la cyclopentadiméthylsiloxane, la cyclohexadiméthylsiloxane, le méthylhexyldiméthylsiloxane ou les isoparaffines telles que les 'ISOPARs', notamment l'isododécane.

Dans un mode particulier de réalisation de l'invention, on choisit le corps gras liquide dans le groupe constitué par :

- les composés liquides non aqueux ayant un paramètre de solubilité global selon l'espace de solubilité de HANSEN inférieur à 17 (MPa)$^{1/2}$,
- ou les monoalcools ayant un paramètre de solubilité global selon l'espace de solubilité de HANSEN inférieur ou égal à 20 (MPa)$^{1/2}$,
- ou leurs mélanges,

Le paramètre de solubilité global $\delta$ global selon l'espace de solubilité de HANSEN est défini dans l'article "Solubility parameter values" de Eric A. Grulke de l'ouvrage "Polymer Handbook" 3$^{\text{ème}}$ édition, Chapitre VII, pages 519-559 par la

relation :

$$\delta = (d_D{}^2 + d_P{}^2 + d_H{}^2)^{1/2}$$

dans laquelle

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.).

La définition des solvants dans l'espace de solubilité tridimensionnel selon HANSEN est décrite dans l'article de C. M. HANSEN : "The three dimensional solubility parameters" J. Paint Technol. 39, 105 (1967).

Parmi les corps gras liquides ayant un paramètre de solubilité global selon l'espace de solubilité de HANSEN inférieur ou égal à 17 (MPa)$^{1/2}$, on peut citer des huiles végétales formées par des esters d'acides gras et de polyols, en particulier les triglycérides, telles que l'huile de tournesol, de sésame ou de colza, ou les esters dérivés d'acides ou d'alcools à longue chaîne (c'est à dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle. On peut également citer les hydrocarbures et notamment des huiles de paraffine, de vaseline, ou le polyisobutylène hydrogéné, l'isododécane, ou encore les 'ISOPARs', isoparaffines volatiles. On peut encore citer les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines, et les huiles siliconées volatiles, notamment cycliques. On peut également citer les solvants, seuls ou en mélange, choisis parmi (i) les esters linéaires, ramifiés ou cycliques, ayant plus de 6 atomes de carbone; (ii) les éthers ayant plus de 6 atomes de carbone, (iii) les cétones ayant plus de 6 atomes de carbone. Par monoalcools ayant un paramètre de solubilité global selon l'espace de solubilité de HANSEN inférieur ou égal à 20 (MPa)$^{1/2}$, on entend les alcools gras aliphatiques ayant au moins 6 atomes de carbone, la chaîne hydrocarbonée ne comportant pas de groupement de substitution. Comme monoalcools selon l'invention, on peut citer l'alcool oléique, le décanol, le dodécanol, l'octadécanol et l'alcool linoléique.

Le choix du milieu non aqueux est effectué par l'homme du métier en fonction de la nature des monomères constituant le polymère et/ou de la nature du stabilisant, comme indiqué ci-après.

D'une manière générale, la dispersion selon l'invention peut être préparée de la manière suivante, donnée à titre d'exemple.

La polymérisation peut être effectuée en dispersion, c'est-à-dire par précipitation du polymère en cours de formation, avec protection des particules formées avec un stabilisant.

On prépare donc un mélange comprenant les monomères initiaux ainsi qu'un amorceur radicalaire. Ce mélange est dissous dans un solvant appelé, dans la suite de la présente description, 'solvant de synthèse'.

Lorsque le corps gras est une huile non volatile, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit donc un solvant de synthèse tel que les monomères initiaux, et l'amorceur radicalaire, y sont solubles, et les particules de polymère obtenu y sont insolubles afin qu'elles y précipitent lors de leur formation.

En particulier, on peut choisir le solvant de synthèse parmi les alcanes tels que l'heptane ou le cyclohexane.

Lorsque le corps gras choisi est une huile volatile, on peut directement effectuer la polymérisation dans ladite huile qui joue donc également le rôle de solvant de synthèse. Les monomères doivent également y être solubles, ainsi que l'amorceur radicalaire, et le polymère obtenu doit y est insoluble.

Les monomères sont de préférence présents dans le solvant de synthèse, avant polymérisation, à raison de 5-20% en poids. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

Les particules de polymère sont stabilisées en surface, lorsqu'elles se forment lors de la polymérisation, grâce à un stabilisant qui peut être un polymère séquencé, un polymère greffé, et/ou un polymère statistique, seul ou en mélange.

La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation. Toutefois, il est également possible de l'ajouter en continu, notamment lorsque l'on ajoute également les monomères en continu.

On peut utiliser 2-30% en poids de stabilisant par rapport au mélange initial de monomères, et de préférence 5-20% en poids.

Lorsque l'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble.

La partie insoluble dudit polymère-stabilisant vient alors s'adsorber à la surface des particules de polymère formées lors de la polymérisation.

Le polymère-stabilisant utilisé lors de la polymérisation doit être soluble, ou dispersible, dans le solvant de synthèse et dans le corps gras.

De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée; les polymères hydrocarbonés greffés avec une chaîne siliconée; les copolymères greffés ayant par exemple un squelette insoluble de type polyacrylique avec des greffons solubles de type acide polyhydroxystéarique.

Comme copolymères blocs greffés ou séquencés comprenant au moins un bloc de type polyorganosiloxane et au moins un bloc d'un polymère radicalaire, on peut citer les copolymères greffés de type acrylique/silicone qui peuvent être employés notamment lorsque le milieu non aqueux est siliconé.

Comme copolymères blocs greffés ou séquencés comprenant au moins un bloc de type polyorganosiloxane et au moins d'un polyéther, on peut utiliser les copolyol diméthicones tels que ceux vendu sous la dénomination "DOW CORNING 3225C" par la société DOW CORNING, les lauryl méthicones tels que ceux vendu sous la dénomination "DOW CORNING Q2-5200 par la société "DOW CORNING".

Comme copolymères d'acrylates ou de méthacrylates d'alcools en C1-C4, et d'acrylates ou de méthacrylates d'alcools en C8-C30, on peut utiliser le copolymère méthacrylate de stéaryle / méthacrylate de méthyle.

Comme copolymères blocs greffés ou séquencés comprenant au moins un bloc résultant de la polymérisation de diènes, hydrogéné ou non hydrogéné,

et au moins un bloc d'un polymère vinylique, on peut citer les copolymères séquencés, notamment de type 'dibloc' ou 'tribloc' du type polystyrène/polyisoprène, polystyrène/polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène).

Comme copolymères blocs greffés ou séquencés comprenant au moins un bloc résultant de la polymérisation de diènes, hydrogéné ou non hydrogéné, et au moins un bloc d'un polymère acrylique, on peut citer les copolymères bi-ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation de diènes, hydrogéné ou non hydrogéné, et au moins un bloc d'un polyéther, on peut citer les copolymères bi- ou triséquencés polyoxyéthylène/polybutadiène ou polyoxyéthylène/polyisobutylène.

Lorsque l'on utilise un polymère statistique en tant que stabilisant, on le choisit de manière à ce qu'il possède une quantité suffisante de groupements le rendant soluble dans le solvant de synthèse envisagé.

On peut ainsi employer des copolymères d'acrylates ou de méthacrylates d'alcools en C1-C4, et d'acrylates ou de méthacrylates d'alcools en C8-C30. On peut en particulier citer le copolymère méthacrylate de stéaryle / méthacrylate de méthyle.

Lorsque le solvant de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale.

En effet, les séquences ou greffons insolubles dans le solvant de synthèse apportent une couverture plus volumineuse à la surface des particules.

D'autre part, lorsque le corps gras liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester.

Lorsque le corps gras liquide ne comprend pas une huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alcools en $C_1$-$C_4$, et d'acrylates ou de méthacrylates d'alcools en $C_8$-$C_{30}$,
- (c) les copolymères blocs greffés ou séquencés comprenant au moins un bloc résultant de la polymérisation de diènes, hydrogéné ou non hydrogéné,

et au moins un bloc d'un polymère vinylique ou acrylique ou d'un polyéther ou d'un polyester, ou leurs mélanges.

Les dispersion obtenues selon l'invention peuvent alors être utilisées dans une composition notamment cosmétique, pharmaceutique et/ou hygiénique, telle qu'une composition de soin ou de maquillage de la peau ou des matières kératiniques, ou encore une composition capillaire ou une composition solaire.

Suivant l'application, on pourra choisir d'utiliser des dispersion de polymères filmifiables ou non filmifiables, dans des huiles volatiles ou non volatiles.

La composition selon l'invention peut comprendre, selon le type d'application envisagé, les constituants classiquement utilisées dans les domaines considérés, qui sont présents en une quantité appropriée à la forme galénique souhaitée.

En particulier, elle peut comprendre des composés pulvérulents et/ou, outre le corps gras liquide de la dispersion, des corps gras additionnels qui peuvent être choisis parmi les cires, les huiles, les gommes et/ou les corps gras pâteux, d'origine végétale, animale, minérale ou de synthèse, voire siliconée.

Il est toutefois possible de ne pas ajouter d'autres corps gras, en particulier lorsque l'huile utilisée pour la dispersion de particules est en quantité suffisante dans le cadre de l'application envisagée.

Parmi les cires susceptibles d'être présentes dans la composition selon l'invention, on peut citer les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de montan; les ozokérites; les cires de polyéthylène; les cires obtenues par synthèse de Fischer-Tropsch; les huiles hydrogénées, les esters gras et les glycérides concrets à 25°C. On peut également utiliser des cires de silicone, parmi lesquelles on peut citer les alkyls, alcoxys et/ou esters de polyméthylsiloxane. Les cires peuvent se présenter sous forme de dispersions stables de particules colloïdales de cire telles qu'elles peuvent être préparées selon des méthodes connues, telles que "Microemulsions Theory and Practice", L. M. Prince Ed., Academic Press (1977), pages 21-32.

Les cires peuvent être présentes à raison de 5-50% en poids dans la composition, notamment lorsque ladite composition trouve une application en tant que mascara.

Parmi les huiles susceptibles d'être présentes dans la composition selon l'invention, on peut citer les huiles hydrocarbonées telles que l'huile de paraffine ou de vaseline; le perhydrosqualène; l'huile d'arara; l'huile d'amande douce, de calophyllum, de palme, de ricin, d'avocat, de jojoba, d'olive ou de germes de céréales; des esters d'acide lanolique, d'acide oléique, d'acide laurique, d'acide stéarique; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones. On peut également utiliser des huiles volatiles, telles que la cyclotétradiméthylsiloxane, la cyclopentadiméthylsiloxane, la cyclohexadiméthylsiloxane, le méthylhexyldiméthylsiloxane ou les isoparaffines.

En particulier, pour faciliter l'application de la composition, on peut y introduire des corps gras volatils.

Lorsque la composition selon l'invention trouve une application en tant que poudre compactée, la phase grasse, usuellement appelée liant, peut de préférence être présente dans la composition à raison de 2-20% en poids.

La composition peut également comprendre des composés pulvérulents, par exemple à raison de 0-98% en poids, et qui peuvent être choisis parmi les pigments et/ou les nacres et/ou les charges habituellement utilisés dans les compositions cosmétiques.

Les pigments peuvent être blancs ou colorés, minéraux et/ou organiques.

On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique.

Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon, de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydrocarbonate de magnésium, l'hydroxyapatite, les microsphères de silice creuses (SILICA BEADS de MAPRECOS), les microcapsules de verre ou de céramique; les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre tout additif usuellement utilisé dans de telles compositions, tel que des épaississants, des antioxydants, des parfums, des conservateurs, des actifs cosmétiques, des hydratants, des vitamines, des acides gras essentiels, des sphingolipides, des filtres solaires, des tensioactifs, des polymères liposolubles comme les polyalkylènes, notamment le polybutène, les polyacrylates et les polymères siliconés compatibles avec les corps gras. Bien entendu l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

Lorsque l'application envisagée pour la composition est une application en tant que mascara, la composition selon la présente invention peut se présenter notamment sous forme de suspension, de dissolution ou de microdispersion de cires en milieu solvant, sous forme solide ou pâteuse anhydre, ou encore sous la forme d'un gel huileux. On a en effet constaté que l'utilisation d'une composition selon l'invention, comprenant des corps gras et des composés pulvérulents, et une dispersion de particules de polymère stabilisé en surface dans un corps gras, permet d'obtenir un gainage adéquat du cil, qui se traduit par un allongement conséquent dudit cil, et une bonne rémanence de la composition à l'eau.

Dans un autre mode de réalisation de l'invention, les compositions selon l'invention peuvent se présenter sous forme d'un produit coulé et comprendre au moins une cire, et une dispersion de particules de polymère réticulé et stabilisé en surface, dans un corps gras liquide cosmétiquement, dermatologiquement, hygiéniquement ou pharmaceutiquement acceptable. Les compositions peuvent alors se présenter sous la forme d'un stick ou bâton, ou sous la forme d'une pâte souple, de viscosité dynamique à 25°C de l'ordre de 3-30 Pa.s. Elles peuvent se présenter sous la forme de produit coulé, préparés de manière usuelle par l'homme du métier, ou encore sous la forme de coupelle utilisable par contact direct ou à l'éponge. En particulier, elles trouvent une application en tant que fond de teint coulé, fard à joues ou à paupières coulé, rouge à lèvres ou d'une base de soin pour les lèvres, ou baume de soin.

Lorsqu'elle se présente sous forme de poudre, notamment compactée, la composition selon l'invention peut être préparée par l'homme du métier de manière usuelle, et notamment par mélange des différents constituants et compactage à l'aide d'une presse mécanique. La composition ainsi obtenue a donc l'aspect d'une poudre compactée, par exemple sous forme de coupelle, de stick, de cylindre, ou sous toute autre forme complexe. La composition selon l'invention peut se présenter sous forme d'une composition pharmaceutique ou hygiénique telle qu'une poudre pour le corps, une poudre pour bébé, ou une poudre antitranspirante. Elle peut également se présenter sous la forme d'un produit de maquillage, tel qu'un fard à joues ou à paupières, un Blush ou une poudre pour le visage.

Les compositions de l'invention peuvent encore se présenter notamment sous forme de gel huileux, de liquide huileux ou huile, de pâte ou de stick, d'aérosol, ou encore sous forme de dispersion vésiculaire contenant des lipides ioniques et/ou non ioniques. Ces formes galéniques sont préparées selon les méthodes usuelles des domaines considérés. Ces compositions à application topique peuvent constituer notamment une composition cosmétique, dermatologique, hygiénique ou pharmaceutique de protection, de traitement ou de soin pour le visage, pour le cou, pour les mains ou pour le corps (par exemple crème de soin anhydre, huile solaire, gel corporel), une composition de maquillage (par exemple gel de maquillage) ou une composition de bronzage artificiel.

L'invention est illustrée plus en détail dans les exemples suivants.

Les exemples 1 à 6 décrivent la préparation de plusieurs dispersions de particules de polymères dans une huile.

Les exemples 7 à 12 décrivent des compositions de maquillage comprenant une dispersion selon l'invention.

## Exemple 1

On mélange 360 g de n-heptane et 15 g de polymère stabilisant séquencé de type copolymère dibloc polystyrène/copoly(éthylène-propylène) vendu sous la dénomination KRATON G1701 (Shell).

On chauffe le mélange pendant au moins 3 h, à environ 60°C afin d'obtenir une solution dispersée.

A 25°C, on ajoute au mélange 19 g de méthacrylate de méthyle, 1 g de diméthacrylate d'éthylène glycol, 0,4 g de tertiobutylperoxy-2-éthylhexanoate (Trigonox 21S de Akzo) et 5 g d'heptane.

On chauffe le mélange à 75°C, sous azote, pendant au moins 3 heures.

On ajoute ensuite, à 75°C et pendant 1,5 heure, un mélange de 76 g de méthacrylate de méthyle, 4 g de diméthacrylate d'éthylène glycol, 1,6 g de tertiobutylperoxy-2-éthylhexanoate (Trigonox 21S de Akzo) et 80 g d'heptane.

A la fin de l'ajout, on chauffe à 85°C pendant 4 heures, on ajoute 1 g de Trigonox dissous dans 5 g d'heptane, et on chauffe encore à 85°C pendant 7 heures.

On obtient une dispersion stable d'aspect laiteux, avec un taux de matière sèche de 18,6% en poids.

La mesure de la taille des particules, effectuée par diffusion quasi-élatique de la lumière avec un Coulter N4 SD, donne les résultats suivants :

- taille moyenne des particules : 160 nm
- polydispersité : inférieure à 0,1.

On mélange 50 g de la dispersion dans l'heptane ci-dessus avec 28,5 g d'huile de paraffine non volatile. On évapore sélectivement l'heptane à l'aide d'un évaporateur rotatif.

On obtient alors une dispersion stable d'aspect laiteux, ayant un taux de matière sèche de 25% en poids, de polyméthacrylate de méthyle réticulé par le diméthacrylate d'éthylène glycol, dans une huile de paraffine.

### Exemple 2

On prépare une dispersion de polyméthacrylate de méthyle réticulé par le diméthacrylate d'éthylène glycol, dans une huile de paraffine ramifiée et volatile (ISOPAR L de Exxon), selon la méthode de l'exemple 1 en remplaçant l'heptane par ladite huile de paraffine ISOPAR L.

On obtient ainsi une dispersion stable, ayant un taux de matière sèche de 19% en poids et une taille moyenne des particules de 159 nm (polydispersité : 0,05).

### Exemple 3

On mélange 20 g de la dispersion dans l'ISOPAR ci-dessus avec 16,2 g de cyclotétradiméthylsiloxane (huile de silicone volatile).

On obtient alors une dispersion stable d'aspect laiteux, constituée de 3,8 g de polyméthacrylate de méthyle réticulé par le diméthacrylate d'éthylène glycol, 16,2 g d'huile de paraffine volatile et 16,2 g d'huile de silicone volatile.

### Exemple 4

On mélange 20 g de la dispersion dans l'ISOPAR de l'exemple 2 avec 16,2 g de benzoate d'alcools en $C_{12}$-$C_{15}$ (FINSOLV TN de Witco).

On obtient alors une dispersion stable d'aspect laiteux, constituée de 3,8 g de polyméthacrylate de méthyle réticulé par le diméthacrylate d'éthylène glycol, 16,2 g d'huile de paraffine volatile et 16,2 g d'ester non volatil.

### Exemple 5

On mélange 360 g de n-heptane et 15 g de polymère stabilisant séquencé de type copolymère dibloc polystyrène/copoly(éthylène-propylène) vendu sous la dénomination KRATON G1701 (Shell).

On chauffe le mélange pendant au moins 3 h, à environ 60°C afin d'obtenir une solution dispersée.

A 25°C, on ajoute au mélange 20 g d'acrylate de méthyle, 0,4 g de tertiobutylperoxy-2-éthylhexanoate et 5 g d'heptane. On chauffe le mélange à 75°C, sous azote, pendant 3 heures. On ajoute à 75°C et pendant 1,5 heures, un mélange de 80 g d'acrylate de méthyle, 1,6 g de tertiobutylperoxy-2-éthylhexanoate et 80 g d'heptane.

A la fin de l'ajout, on chauffe à 85°C pendant 4 heures, puis on ajoute 1 g de Trigonox dissous dans 5 g d'heptane, et on chauffe encore à 85°C pendant 7 heures.

On obtient une dispersion stable d'aspect laiteux, avec un taux de matière sèche de 19% en poids.

La mesure de la taille des particules, effectuée par diffusion quasi-élatique de la lumière avec un Coulter N4 SD, donne les résultats suivants :

. taille moyenne des particules : 230 nm
. polydispersité : inférieure à 0,1.

On mélange 50 g de la dispersion dans l'heptane ci-dessus avec 28,5 g d'huile de paraffine non volatile. On évapore sélectivement l'heptane à l'aide d'un évaporateur rotatif.

On obtient alors une dispersion stable d'aspect laiteux, ayant un taux de matière sèche de 25% en poids, de polyacrylate de méthyle (Tg = 10°C) dans une huile de paraffine non volatile.

### Exemple 6

On prépare une dispersion de polyacrylate de méthyle dans une huile de paraffine ramifiée et volatile (ISOPAR L de Exxon), de la même manière que dans l'exemple 5, en remplaçant l'heptane par ladite huile de paraffine ISOPAR L.

On obtient ainsi une dispersion stable, ayant un taux de matière sèche de 20% en poids et une taille moyenne des particules de 197 nm (polydispersité : 0,06).

Cette dispersion est filmogène et donne, après séchage, un film continu et transparent.

### Exemple 7 : mascara waterproof

On prépare la composition de la manière suivante : les composants de la phase A sont fondus, on y ajoute les pigments et on mélange. Les composants de la phase B sont mélangés et sont ajoutés aux composants de la phase A.

| Phase A | |
|---|---|
| . Cire de paraffine | 12 g |
| . Alcool de lanoline | 15 g |
| . Oxyde de fer noir | 5 g |

| Phase B | |
|---|---|
| . Montmorillonite | 8 g |
| . Amidon | 2 g |
| . Isoparaffine | 45 g |
| . Dispersion selon l'exemple 1 | 5 g |

On obtient un mascara ayant de bonnes propriétés cosmétiques.

Exemple 8

On prépare un rouge à lèvres sous forme de pâte molle ayant la composition suivante :

| . cires (abeilles et Carnauba) | 5 g |
|---|---|
| . huiles | 20 g |
| . lanoline acétylée | 20 g |
| . lanoline | 30 g |
| . charges | 10 g |
| . pigments | 10 g |
| . dispersion selon l'exemple 1 | 5 g |

La composition est préparée par chauffage des différents ingrédients à 95-100°C, en mélangeant de manière à obtenir un mélange parfaitement homogène.
Après refroidissement, on obtient un rouge à lèvres facile à appliquer, et qui permet l'obtention d'un film agréable à porter.
On constate que le film ne migre pas dans les ridules de la peau, même après avoir été porté pendant plusieurs heures.

Exemple 9

On prépare un rouge à lèvres sous forme d'un stick, ayant la composition suivante:

| | |
|---|---|
| . huile de ricin | 15 g |
| . huile de jojoba | 13 g |
| . huile de coco hydrogénée | 7 g |
| . lanolate d'isopropyle | 25 g |
| . cire de Carnauba | 10 g |
| . cire de polyéthylène | 10 g |
| . pigments et charges | 15 g |
| . dispersion de l'exemple 1 | 5 g |

Le bâton est préparé de la façon suivante : on chauffe la phase grasse à une température d'environ 100°C, on ajoute les charges et pigments, puis l'on mélange le tout à l'aide d'une turbine Moritz à la vitesse de 3000 tr/min. On peut alors couler ledit mélange dans des moules adéquats.

La composition obtenue est aisée et agréable à appliquer. On constate que le film ne migre pas dans les ridules de la peau, même après avoir été porté pendant plusieurs heures.

Exemple 10

On prépare une poudre compactée ayant la composition suivante :

| Composition A : | |
|---|---|
| - Talc | 30 g |
| - Oxychlorure de bismuth | 10 g |
| - Stéarate de zinc | 4 g |
| - Poudre de Nylon | 20 g |
| - Dispersion de l'exemple 5 | 5 g |

| Composition B : | |
|---|---|
| - Oxydes de fer | 2 g |
| - Huile de vaseline | 6 g |

La poudre est obtenue de la façon suivante : on broie la composition A dans un broyeur de type KENWOOD pendant environ 5 minutes sous faible agitation, on ajoute la composition B et on broie l'ensemble environ 2 minutes à la même vitesse, puis 3 minutes à une vitesse plus rapide. On tamise ensuite la préparation sur un tamis de 0,16 mm, puis on compacte ce mélange dans des coupelles.

On obtient une poudre compactée présentant une bonne adhésion, qui s'étale bien et de manière agréable sur la peau, tout en étant douce au toucher.

Exemple 11 : gel pour le visage

On prépare la composition suivante :

| . isopropyl palmitate | 10 g |
| . vaseline (cire) | 5 g |
| . hectorite modifiée (argile) | 0,15 g |
| . ozokérite (cire) | 5 g |
| . septaoléate de sorbitane oxyéthyléné (40OE) | 5 g |
| . dispersion de l'exemple 1 (25% de matière sèche) | 75 g |

On obtient un gel ayant de bonnes propriétés cosmétiques.

Exemple 12 : huile de soin

On prépare la composition suivante :

| . dispersion de l'exemple 2 (25% de matière sèche) | 70 g |
| . huile de jojoba | 15 g |
| . huile de soja | 15 g |

On obtient une huile de soin qui peut être appliquée sur le corps ou le visage.

**Revendications**

1. Composition cosmétique, comprenant des corps gras et des composés pulvérulents, caractérisée par le fait qu'elle comprend une dispersion de particules de polymère stabilisé en surface dans un corps gras liquide.

2. Composition se présentant sous forme d'un produit coulé et comprenant au moins une cire, caractérisée par le fait qu'elle comprend en outre une dispersion de particules de polymère réticulé et stabilisé en surface, dans un corps gras liquide cosmétiquement, dermatologiquement, hygiéniquement ou pharmaceutiquement acceptable.

3. Composition se présentant sous forme d'une poudre compacte, comprenant un liant gras et des composés pulvérulents, caractérisée par le fait qu'elle comprend une dispersion de particules de polymère stabilisé en surface dans un corps gras liquide cosmétiquement, dermatologiquement, pharmaceutiquement ou hygiéniquement acceptable.

4. Composition comprenant une dispersion de particules de polymère non filmogène et stabilisé en surface, dans un corps gras liquide non volatil et cosmétiquement, dermatologiquement, hygiéniquement ou pharmaceutiquement acceptable, ladite dispersion ayant un taux de matière sèche d'au moins 15% en poids.

5. Composition selon l'une des revendications précédentes, dans laquelle le polymère est choisi parmi les polymères radicalaires, les polycondensats, les polymères d'origine naturelle et leur mélange.

6. Composition selon l'une des revendications précédentes, dans laquelle le corps gras liquide est constitué d'huiles d'origine minérale, animale, végétale ou synthétique, carbonées, hydrocarbonées, fluorées et/ou siliconées, seules ou en mélange.

7. Composition selon l'une des revendications précédentes, dans laquelle le corps gras liquide est choisi parmi l'huile de paraffine ou de vaseline; l'huile de vison, l'huile de tortue, l'huile de soja, le perhydrosqualène; l'huile d'amande douce, de calophyllum, de palme, l'huile de pépins de raisin, l'huile de sésame, l'huile de maïs, l'huile de colza, l'huile de tournesol, l'huile de coton, de ricin, d'avocat, de jojoba, d'olive ou de germes de céréales; des esters d'acide lanolique, d'acide oléique, d'acide laurique, d'acide stéarique; les esters gras, tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine; les acides gras supérieurs tels que l'acide myristique, l'acide palmitique, l'acide stéarique, l'acide béhénique, l'acide oléique, l'acide linoléique, l'acide linolénique ou l'acide isostéarique; les alcools gras supérieurs tels que le cétanol, l'alcool stéarylique ou l'alcool oléique, l'alcool linoléique ou linolénique, l'alcool isostéarique ou l'octyl dodécanol; les huiles siliconées telles que les PDMS, éventuellement phénylées telles que les phényltriméthicones ou éventuellement substitués par des groupements aliphatiques et/ou aromatiques, éventuellement fluorés, ou par des groupements fonctionnels tels que des groupements hydroxyles, thiols et/ou amines.; les polysiloxanes modifiés par des acides gras, des alcools gras ou des polyoxyalkylènes, les silicones fluorées, les huiles perfluorées; les huiles volatiles, telles que la cyclotétradiméthylsiloxane, la cyclopentadiméthylsiloxane, la cyclohexadiméthylsiloxane, le méthylhexyldiméthylsiloxane ou les isoparaffines telles que les 'ISOPARs', notamment l'isododécane.

8. Composition selon l'une des revendications précédentes, dans laquelle le corps gras liquide est choisi dans le groupe constitué par :

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges,

9. Composition selon l'une des revendications précédentes, dans laquelle les particules de polymère sont stabilisées en surface grâce à un stabilisant choisi parmi un polymère séquencé, un polymère greffé, et/ou un polymère statistique, seul ou en mélange.

10. Composition selon la revendication 9, dans laquelle le stabilisant est choisi parmi les polymères siliconés greffés avec une chaîne hydrocarbonée; les polymères hydrocarbonés greffés avec une chaîne siliconée; les copolymères greffés ayant par exemple un squelette insoluble de type polyacrylique avec des greffons solubles de type acide polyhydroxystéarique; les copolymères blocs greffés ou séquencés comprenant au moins un bloc de type polyorganosiloxane et au moins un bloc d'un polymère radicalaire; les copolymères blocs greffés ou séquencés comprenant au moins un bloc de type polyorganosiloxane et au moins d'un polyéther; les copolymères d'acrylates ou de méthacrylates d'alcools en C1-C4, ou d'acrylates ou de méthacrylates d'alcools en C8-C30; les copolymères blocs greffés ou séquencés comprenant au moins un bloc résultant de la polymérisation de diènes et au moins un bloc d'un polymère vinylique; les copolymères blocs greffés ou séquencés comprenant au moins un bloc résultant de la polymérisation de diènes et au moins un bloc d'un polymère acrylique; les copolymères blocs greffés ou séquencés comprenant au moins un bloc résultant de la polymérisation de diènes et au moins un bloc d'un polyéther.

11. Composition selon l'une des revendications précédentes, comprenant en outre des composés pulvérulents et/ou des corps gras additionnels choisis parmi les cires, les huiles, les gommes et/ou les corps gras pâteux, d'origine végétale, animale, minérale, de synthèse, ou siliconée.

12. Composition selon l'une des revendications précédentes, se présentant sous forme de suspension, de dissolution ou de microdispersion de cires en milieu solvant, sous forme solide ou pâteuse anhydre, ou encore sous la forme d'un gel huileux.

13. Composition selon la revendication 12, se présentant sous la forme d'un mascara.

14. Composition selon l'une des revendications 1 à 11, se présentant sous forme d'un stick ou bâton; sous la forme d'une pâte souple, de viscosité dynamique à 25°C de l'ordre de 3-30 Pa.s; sous la forme de coupelle.

15. Composition selon la revendication 14, se présentant sous la forme d'un produit de soin et/ou de maquillage de la peau, tel qu'un fond de teint coulé, un fard à joues ou à paupières coulé, un rouge à lèvres ou une base de soin pour les lèvres, ou un baume de soin.

16. Composition selon l'une des revendications 1 à 11, se présentant sous forme d'une poudre pour le corps, d'une poudre pour bébé, d'une poudre antitranspirante, d'un produit de maquillage, tel qu'un fard à joues ou à paupières, un Blush ou une poudre pour le visage.

17. Composition selon l'une des revendications 1 à 11, se présentant sous forme de gel huileux, de liquide huileux ou huile, de pâte ou de stick, d'aérosol, de dispersion vésiculaire contenant des lipides ioniques et/ou non ioniques.

**18.** Composition selon la revendication 17, se présentant sous forme d'une composition cosmétique, dermatologique, hygiénique ou pharmaceutique de protection, de traitement ou de soin pour le visage, pour le cou, pour les mains ou pour le corps, une composition antisolaire, une composition de bronzage artificiel.

**19.** Utilisation dans une composition cosmétique selon la revendication 1 pour le maquillage des yeux, comprenant des corps gras et des composés pulvérulents, d'une dispersion de particules de polymère stabilisé en surface dans un corps gras liquide, afin d'améliorer l'allongement des cils et/ou d'améliorer la rémanence à l'eau de la composition.

**20.** Utilisation dans une composition selon la revendication 2 sous forme de produit coulé et comprenant au moins une cire, d'une dispersion de particules de polymère non filmogène et stabilisé en surface dans un corps gras liquide cosmétiquement, dermatologiquement, pharmaceutiquement ou hygiéniquement acceptable, dans le but d'atténuer la migration des constituants de la composition dans les ridules de la peau et/ou dans le but d'améliorer la tenue de la composition.

**21.** Utilisation dans une composition selon la revendication 3 se présentant sous forme d'une poudre compacte, comprenant un liant gras et des composés pulvérulents, d'une dispersion de particules de polymère stabilisé en surface dans un corps gras liquide cosmétiquement, dermatologiquement, pharmaceutiquement ou hygiéniquement acceptable, dans le but de faciliter le compactage de ladite composition.

**22.** Utilisation dans une composition cosmétique selon la revendication 4, dermatologique, pharmaceutique ou hygiénique, d'une dispersion de particules de polymère non filmogène et stabilisé en surface, dans un corps gras liquide non volatil et cosmétiquement, dermatologiquement, hygiéniquement ou pharmaceutiquement acceptable, ladite dispersion ayant un taux de matière sèche d'au moins 15% en poids, dans le but d'estomper les rides et/ou les ridules de la peau.

## Claims

**1.** Cosmetic composition comprising fatty substances and pulverulent compounds, characterized in that it comprises a dispersion of surface-stabilized polymer particles in a liquid fatty substance.

**2.** Composition in the form of a cast product comprising at least one wax, characterized in that it also comprises a dispersion of crosslinked and surface-stabilized polymer particles in a cosmetically, dermatologically, hygienically or pharmaceutically acceptable liquid fatty substance.

**3.** Composition in the form of a compact powder, comprising a fatty binder and pulverulent compounds, characterized in that it comprises a dispersion of surface-stabilized polymer particles in a cosmetically, dermatologically, pharmaceutically or hygienically acceptable liquid fatty substance.

**4.** Composition comprising a dispersion of non-film-forming surface-stabilized polymer particles in a cosmetically, dermatologically, hygienically or pharmaceutically acceptable non-volatile liquid fatty substance, the said dispersion having a solids content of at least 15 % by weight.

**5.** Composition according to one of the preceding claims, in which the polymer is chosen from radical polymers, polycondensates, polymers of natural origin and a mixture thereof.

**6.** Composition according to one of the preceding claims, in which the liquid fatty substance consists of carbon-based, hydrocarbon, fluoro and/or silicone oils of mineral, animal, plant or synthetic origin, alone or as a mixture.

**7.** Composition according to one of the preceding claims, in which the liquid fatty substance is chosen from liquid paraffin or liquid petroleum jelly; mink oil, turtle oil, soya oil, perhydrosqualene; sweet almond oil, calophyllum oil, palm oil, grapeseed oil, sesame oil, corn oil, rapeseed oil, sunflower oil, cotton oil, castor oil, avocado oil, jojoba oil, olive oil or cereal germ oil; esters of lanolic acid, of oleic acid, of lauric acid or of stearic acid; fatty esters such as isopropyl myristate, isopropyl palmitate, butyl stearate, hexyl laurate, diisopropyl adipate, isononyl isononate, 2-ethylhexyl palmitate, 2-hexyldecyl laurate, 2-octyldecyl palmitate, 2-octyldodecyl myristate or lactate, 2-diethylhexyl succinate, diisostearyl malate, glyceryl or diglyceryl triisostearate; higher fatty acids such as myristic acid, palmitic acid, stearic acid, behenic acid, oleic acid, linoleic acid, linolenic acid or isostearic acid; higher fatty alcohols such as cetanol, stearyl alcohol or oleyl alcohol, linoleyl or linolenyl alcohol, isostearyl alcohol or octyldodecanol; silicone oils such as PDMSs, which are optionally phenylated, such as phenyltrimethicones, or which are optionally substituted with aliphatic and/or aromatic groups, which are optionally fluorinated, or substituted with functional groups such as hydroxyl, thiol and/or amine groups; polysiloxanes modified with fatty acids, with fatty alcohols or with polyoxyalkylenes, fluoro silicones and perfluoro oils; volatile oils such as cyclotetradimethylsiloxane, cyclopentadimethylsiloxane, cyclohexadimethylsiloxane and methylhexyldimethylsiloxane or isoparaffins such as "ISOPARs", in particular isododecane.

8. Composition according to one of the preceding claims, in which the liquid fatty substance is chosen from the group consisting of:

- non-aqueous liquid compounds having a global solubility parameter according to the Hansen solubility space of less than 17 $(MPa)^{1/2}$,
- or monoalcohols having a global solubility parameter according to the Hansen solubility space of less than or equal to 20 $(MPa)^{1/2}$,
- or mixtures thereof.

9. Composition according to one of the preceding claims, in which the polymer particles are surface-stabilized by means of a stabilizer chosen from a sequential polymer, a grafted polymer and/or a random polymer, alone or as a mixture.

10. Composition according to Claim 9, in which the stabilizer is chosen from silicone polymers grafted with a hydrocarbon chain; hydrocarbon polymers grafted with a silicone chain; grafted copolymers having, for example, an insoluble skeleton of polyacrylic type with soluble grafts of polyhydroxystearic acid type; sequential or grafted block copolymers comprising at least one block of polyorganosiloxane type and at least one block of a radical polymer; sequential or grafted block copolymers comprising at least one block of polyorganosiloxane type and at least one polyether; copolymers acrylates or methacrylates of C1-C4 alcohols or of acrylates or methacrylates of C8-C30 alcohols; sequential or grafted block copolymers comprising at least one block resulting from the polymerization of dienes and at least one block of a vinyl polymer; sequential or grafted block copolymers comprising at least one block resulting from the polymerization of dienes and at least one block of an acrylic polymer; sequential or grafted block copolymers comprising at least one block resulting from the polymerization of dienes and at least one block of a polyether.

11. Composition according to one of the preceding claims, also comprising pulverulent compounds and/or additional fatty substances chosen from waxes, oils, gums and/or pasty fatty substances, which are of plant, animal, mineral or synthetic origin or which are silicone-based.

12. Composition according to one of the preceding claims, in the form of a suspension, a solution or a microdispersion of waxes in a solvent medium, in anhydrous solid or pasty form, or alternatively in the form of an oily gel.

13. Composition according to Claim 12, in the form of a mascara.

14. Composition according to one of Claims 1 to 11, in the form of a stick or pencil; in the form of a flexible paste, with a dynamic viscosity at 25°C of about 3-30 Pa·s; in the form of a cupel.

15. Composition according to Claim 14, in the form of a product to care for and/or make up the skin, such as a cast foundation, a cast make-up rouge or eyeshadow, a lipstick or a care base for the lips, or a care balm.

16. Composition according to one of Claims 1 to 11, in the form of a body powder, a baby powder, an antiperspirant powder or a make-up product such as a make-up rouge or eyeshadow, a blusher or a face powder.

17. Composition according to one of Claims 1 to 11, in the form of an oily gel, an oily liquid or an oil, a paste or stick or an aerosol, or of a vesicle dispersion containing ionic and/or nonionic lipids.

18. Composition according to Claim 17, in the form of a cosmetic, dermatological, hygiene or pharmaceutical composition for protection, treatment or care of the face, the neck, the hands or the body, an antisun composition or an artificial tanning composition.

19. Use, in a cosmetic composition according to Claim 1 for making up the eyes, comprising fatty substances and pulverulent compounds, of a dispersion of surface-stabilized polymer particles in a liquid fatty substance, in order to improve the lengthening of the eyelashes and/or to improve the remanence in water of the composition.

**20.** Use, in a composition according to Claim 2 in the form of a cast product comprising at least one wax, of a dispersion of non-film-forming surface-stabilized polymer particles in a cosmetically, dermatologically, pharmaceutically or hygienically acceptable liquid fatty substance, for the purpose of attenuating the migration of the constituents of the composition into the fine lines in the skin and/or for the purpose of improving the staying power of the composition.

**21.** Use, in a composition according to Claim 3 in the form of a compact powder comprising a fatty binder and pulverulent compounds, of a dispersion of surface-stabilized polymer particles in a cosmetically, dermatologically, pharmaceutically or hygienically acceptable liquid fatty substance, for the purpose of facilitating the compacting of the said composition.

**22.** Use, in a cosmetic, dermatological, pharmaceutical or hygiene composition according to Claim 4, of a dispersion of non-film-forming surface-stabilized polymer particles in a cosmetically, dermatologically, hygienically or pharmaceutically acceptable non-volatile liquid fatty substance, the said dispersion having a solids content of at least 15 % by weight, for the purpose of softening the wrinkles and/or fine lines in the skin.

**Patentansprüche**

**1.** Kosmetische Zusammensetzung, die Fettsubstanzen und pulverförmige Verbindungen enthält,
dadurch gekennzeichnet, daß
sie eine Dispersion von Partikeln eines an der Oberfläche stabilisierten Polymers in einer flüssigen Fettsubstanz enthält.

**2.** Zusammensetzung, die in Form eines gegossenen Produkts vorliegt und mindestens ein Wachs enthält,
dadurch gekennzeichnet, daß
sie ferner eine Dispersion von Partikeln eines vernetzten und an der Oberfläche stabilisierten Polymers in einer flüssigen kosmetisch, dermatologisch, hygienisch oder pharmazeutisch akzeptablen Fettsubstanz enthält.

**3.** Zusammensetzung, die in Form eines kompakten Pulvers vorliegt und ein Fettbindemittel und pulverförmige Bestandteile enthält,
dadurch gekennzeichnet, daß
sie eine Dispersion von Partikeln eines an der Oberfläche stabilisierten Polymers in einer flüssigen kosmetisch, dermatologisch, pharmazeutisch oder hygienisch akzeptablen Fettsubstanz enthält.

**4.** Zusammensetzung, die eine Dispersion von Partikeln eines nicht filmbildenden und an der Oberfläche stabilisierten Polymers in einer flüssigen nicht flüchtigen und kosmetisch, dermatologisch, hygienisch oder pharmazeutisch akzeptablen Fettsubstanz enthält, wobei die Dispersion einen Trockensubstanzgehalt von mindestens 15 Gew.-% aufweist.

**5.** Zusammensetzung nach einem der vorhergehenden Ansprüche, wobei das Polymer unter den radikalischen Polymeren, Polykondensaten, Polymeren natürlichen Ursprungs und deren Gemischen ausgewählt ist.

**6.** Zusammensetzung nach einem der vorhergehenden Ansprüche, wobei die flüssige Fettsubstanz aus mineralischen, tierischen, pflanzlichen oder synthetischen Ölen, Kohlenstoffölen, Kohlenwasserstoffölen, fluorierten Ölen und/oder Siliconölen oder Gemischen dieser Öle besteht.

**7.** Zusammensetzung nach einem der vorhergehenden Ansprüche, wobei die flüssige Fettsubstanz ausgewählt ist unter: Paraffinöl oder Vaselineöl, Nerzöl, Schildkrötenöl, Sojaöl, Perhydrosqualen, Süßmandelöl, Calophyllumöl, Palmöl, Traubenkernöl, Sesamöl, Maisöl, Rapsöl, Sonnenblumenöl, Öl von Baumwolle, Ricinusöl, Avocadoöl, Jojobaöl, Olivenöl oder Öl von Getreidekeimen, Estern von Lanolinsäure, Ölsäure, Laurinsäure und Stearinsäure, Fettsäureestern, wie Isopropylmyristat, Isopropylpalmitat, Butylstearat, Hexyllaurat, Diisopropyladipat, Isononylisononat, 2-Ethylhexylpalmitat, 2-Hexyldecyllaurat, 2-Octyldecylpalmitat, 2-Octyldodecylmyristat, 2-Octyldodecyllactat, 2-Diethylhexylsuccinat, Diisostearylmalat, Glycerintriisostearat oder Diglycerintriisostearat, höheren Fettsäuren, wie Myristinsäure, Palmitinsäure, Stearinsäure, Behensäure, Ölsäure, Linolsäure, Linolensäure oder Isostearinsäure, höheren Fettalkoholen wie Cetanol, Stearylalkohol, Oleylalkohol, Linoleylalkohol, Linolenylalkohol, Isostearylalkohol oder Octyldodecanol, Siliconölen, wie die PDMS, die ggf. Phenylgruppen enthalten, wie Phenyltrimethicone, oder ggf. mit aliphatischen und/oder aromatischen ggf. fluorierten Gruppen oder mit funktionellen Gruppen, wie Hydroxy-, Thiol- und/oder Aminogruppen, substituiert sind, mit Fettsäuren, Fettalkoholen oder Polyoxyalkylenen modifizierten Polysiloxanen, fluorierten Siliconen, perfluorierten Ölen, flüchtigen Ölen, wie Cyclotetradimethylsiloxan, Cyclopentadimethylsiloxan, Cyclohexadimethylsiloxan, Methylhexadimethylsiloxan oder die Isoparaffine wie die "ISOPARs", insbesondere Isododecan.

8. Zusammensetzung nach einem der vorhergehenden Ansprüche, wobei die flüssige Fettsubstanz ausgewählt ist unter:

   - nicht wäßrigen flüssigen Verbindungen mit einem Gesamtsolubilitätsparameter nach dem Solubilitätsraum von HANSEN unter 17 $(MPa)^{1/2}$,
   - oder Monoalkoholen mit einem Gesamtsolubilitätsparameter nach dem Solubilitätsraum von HANSEN von höchstens 20 $(MPa)^{1/2}$,
   - oder deren Gemischen.

9. Zusammensetzung nach einem der vorhergehenden Ansprüche, wobei die Partikel des Polymers an der Oberfläche durch ein Stabilisierungsmittel stabilisiert sind, das unter Blockpolymeren, Pfropfpolymeren und/oder statistischen Polymeren oder deren Gemischen ausgewählt ist.

10. Zusammensetzung nach Anspruch 9, wobei das Stabilisierungsmittel ausgewählt ist unter: mit einer Kohlenwasserstoffkette gepfropften Siliconpolymeren, mit einer Siliconkette gepfropften Kohlenwasserstoffpolymeren, gepfropften Copolymeren, die beispielsweise ein unlösliches Grundgerüst vom Typ Polyacryl mit löslichen Propfzweigen vom Typ Polyhydroxystearinsäure aufweisen, gepfropften Blockcopolymeren oder sequentiellen Blockcopolymeren, die mindestens einen Block vom Typ Polyorganosiloxan und mindestens einen Block eines radikalischen Polymers aufweisen, gepfropften Blockcopolymeren oder sequentiellen Blockcopolymeren, die mindestens einen Block vom Typ Polyorganosiloxan und mindestens einen Polyetherblock aufweisen, Copolymeren von Acrylaten oder Methacrylaten von $C_{1-4}$-Alkoholen und Acrylaten oder Methacrylaten von $C_{8-30}$-Alkoholen, gepfropften Blockcopolymeren oder sequentiellen Blockcopolymeren, die mindestens einen Block enthalten, der aus der Polymerisation von Dienen stammt, und mindestens einen Block eines Vinylpolymers, gepfropften Blockcopolymeren oder sequentiellen Blockcopolymeren, die mindestens einen Block, der aus der Polymerisation von Dienen stammt, und mindestens einen Block eines Acrylpolymers enthalten, gepfropften Blockcopolymeren oder sequentiellen Blockcopolymeren, die mindestens einen Block, der aus der Polymerisation von Dienen stammt, und mindestens einen Polyetherblock enthalten.

11. Zusammensetzung nach einem der vorhergehenden Ansprüche, die ferner pulverförmige Verbindungen und/oder zusätzliche Fettsubstanzen enthält, die unter den Wachsen, Ölen, Gummen und/oder pastösen Fettsubstanzen pflanzlichen, tierischen, mineralischen, synthetischen Ursprungs oder den siliconhaltigen Verbindungen ausgewählt sind.

12. Zusammensetzung nach einem der vorhergehenden Ansprüche, die in Form einer Suspension, Lösung oder Mikrodispersion von Wachsen in einem Lösungsmittelmedium, in fester oder pastöser wasserfreier Form oder auch in Form eines öligen Gels vorliegt.

13. Zusammensetzung nach Anspruch 12, die in Form von Mascara vorliegt.

14. Zusammensetzung nach einem der Ansprüche 1 bis 11, die in Form eines Sticks oder Stiftes, in Form einer weichen Paste mit einer dynamischen Viskosität bei 25 °C in der Größenordnung von 3 bis 30 Pa•s oder in Form eines Tiegelchens vorliegt.

15. Zusammensetzung nach Anspruch 14, die in Form eines Produkts zur Pflege und/oder zum Schminken der Haut, wie als gegossenes Make-up, gegossenes Wagenrouge oder gegossener Lidschatten, dekorativer oder pflegender Lippenstift oder Pflegebalsam, vorliegt.

16. Zusammensetzung nach einem der Ansprüche 1 bis 11, die in Form eines Puders für den Körper, Babypuders, Antitranspirantpuders, oder als Produkt zum Schminken, wie Wagenrouge oder Lidschatten, Rouge oder Puder für das Gesicht vorliegt.

17. Zusammensetzung nach einem der Ansprüche 1 bis 11, die in Form eines öligen Gels, als ölige Flüssigkeit oder Öl, Paste oder Stick, Aerosol oder Vesikeldispersion, die ionische und/oder nicht ionische Lipide enthält, vorliegt.

18. Zusammensetzung nach Anspruch 17, die in Form einer kosmetischen, dermatologischen, hygienischen oder pharmazeutischen Zusammensetzung zum Schutz, zur Behandlung oder zur Pflege des Gesichts, des Halses, der Hände oder des Körpers, als Sonnenschutzzusammensetzung oder Zusammensetzung zur künstlichen Bräunung vorliegt.

19. Verwendung einer Dispersion von Partikeln eines an der Oberfläche stabilisierten Polymers in einer flüssigen Fettsubstanz in einer kosmetischen Zusammensetzung nach Anspruch 1 zum Schminken der Augen, die Fettsubstanzen und pulverförmige Verbindungen enthält, um die Verlängerung der Wimpern und/oder die Beständigkeit der Zusammensetzung gegenüber Wasser zu verbessern.

20. Verwendung einer Dispersion von Partikeln eines nicht filmbildenden und an der Oberfläche stabilisierten Polymers in einer flüssigen kosmetisch dermatologisch, pharmazeutisch oder hygienisch akzeptablen Fettsubstanz in einer Zusammensetzung nach Anspruch 2, um die Migration der Bestandteile der Zusammensetzung in die Falten der Haut zu verhindern und/oder die Haltbarkeit der Zusammensetzung zu verbessern.

21. Verwendung einer Dispersion von Partikeln eines an der Oberfläche stabilisierten Polymers in einer flüssigen kosmetisch dermatologisch, pharmazeutisch oder hygienisch akzeptablen Fettsubstanz in einer Zusammensetzung nach Anspruch 3, die in Form eines kompakten Pulvers vorliegt und ein Fettbindemittel und pulverförmige Verbindungen enthält, um das Verpressen der Zusammensetzung zu erleichtern.

22. Verwendung einer Dispersion von Partikeln eines nicht filmbildenden und an der Oberfläche stabilisierten Polymers in einer flüssigen nicht flüchtigen und kosmetisch, dermatologisch, hygienisch oder pharmazeutisch akzeptablen Fettsubstanz in einer kosmetischen Zusammensetzung nach Anspruch 4, wobei die Dispersion einen Trockensubstanzgehalt von mindestens 15 Gew.-% aufweist, um die Falten und/oder Fältchen der Haut zu verbergen.